# EUROPÄISCHE PATENTSCHRIFT

(11) **EP 0 648 097 B1**
(45) Veröffentlichungstag und Bekanntmachung des Hinweises auf die Patenterteilung: **05.02.1997**
(21) Anmeldenummer: 93911458.3
(22) Anmeldetag: 13.04.1993
(51) Int. Cl.: A61B 19/00, A61B 1/12, A61L 2/18

(54) **VERFAHREN UND VORRICHTUNG ZUR REINIGUNG ÄRZTLICHER INSTRUMENTE**
METHOD AND DEVICE FOR CLEANING MEDICAL INSTRUMENTS
PROCEDE ET DISPOSITIF DE NETTOYAGE D'INSTRUMENTS MEDICAUX

(30) Priorität: 26.06.1992 DE 4221102
(43) Veröffentlichungstag der Anmeldung: 19.04.1995
(73) Patentinhaber: AESCULAP AG, D-78501 Tuttlingen (DE)
(72) Erfinder: GIORDANO, Nicola, D-7730 Villingen-Schwenningen (DE); WEISSHAUPT, Dieter, D-7717 Immendingen (DE)
(74) Vertreter: Hoeger, Stellrecht & Partner
(86) Internationale Anmeldenummer: EP9300889
(87) Internationale Veröffentlichungsnummer: WO9400068

(56) Entgegenhaltungen:
- EP-A- 0 345 228
- DE-A- 3 232 329
- DE-A- 3 416 743
- GB-A- 1 168 035
- US-A- 3 956 011
- US-A- 4 352 361

## Beschreibung

Die Erfindung betrifft ein Verfahren zur Reinigung ärztlicher Rohrschaftinstrumente, bei dem man die Rohrschaftinstrumente mit einem Ende in eine Reinigungsflüssigkeit eintaucht und am anderen Ende Reinigungsflüssigkeit absaugt. Ferner betrifft die Erfindung eine Vorrichtung zur Reinigung ärztlicher Rohrschaftinstrumente mit einem Behälter für eine Reinigungsflüssigkeit, in die die Rohrschaftinstrumente eintauchen.

Ärztliche Instrumente müssen nach dem Einsatz gründlich gereinigt werden, insbesondere müssen eingetretene Körperflüssigkeiten vollständig aus den Instrumenten entfernt werden, bevor diese sterilisiert werden.

Es hat sich als schwierig erwiesen, eine solche gründliche Vorreinigung bei Rohrschaftinstrumenten durchzuführen, die enge, insbesondere kanalförmige Hohlräume aufweisen, vor allen Dingen dann, wenn in den Hohlräumen zusätzlich noch andere Teile angeordnet sind, beispielsweise Zugstangen, Kabel etc., so daß man mit Reinigungsbürsten nicht in das Innere der Hohlräume eindringen kann. Bei einer bekannten Reinigungsvorrichtung für Rohrschaftinstrumente werden diese dadurch gereinigt, daß sie mit der Spitze voran in einen Köcher eingestellt werden, der durchspült wird (DE 34 16 743 A1). Dazu ist es notwendig, an dem Rohrschaftinstrument ganz spezielle Anschlüsse für Saugschläuche oder Flüssigkeitszufuhrschläuche vorzusehen.

Es ist Aufgabe der Erfindung, ein gattungsgemäßes Verfahren so abzuändern, daß Rohrschaftinstrumente an sich bekannter Bauart vorteilhaft gereinigt werden können, und zwar nach Möglichkeit besonders gründlich.

Diese Aufgabe wird bei einem Verfahren der eingangs beschriebenen Art erfindungsgemäß durch die kennzeichnenden Merkmale des Patentanspruchs 1 gelöst.

Durch die spezielle Anordnung des Rohrschaftinstruments und durch das Aufstecken eines Saugschlauchs auf das vordere Ende des Schafts ist es möglich, Rohrschaftinstrumente zu durchspülen, ohne daß dazu spezielle Saug- oder Spülanschlüsse notwendig werden. Es genügt, den Saugschlauch auf das vordere Ende des Instruments dichtend aufzustecken und dann das hintere Ende mit dem Griffteil oder einem großen Teil des Schafts in die Reinigungsflüssigkeit einzutauchen. Allein durch Anlegen eines Unterdrucks an den Schlauch erreicht man damit eine vollständige Durchspülung des Instruments.

Günstig ist es dabei, wenn man mehrere Rohrschaftinstrumente über jeweils einen Saugschlauch gleichzeitig an eine gemeinsame Saugleitung anschließt und jeden Anschluß zwischen einem Rohrschaftinstrument und der Saugleitung individuell öffnet oder schließt. Dadurch wird es möglich, eine größere Anzahl derartiger Rohrschaftinstrumente mit der gleichen Saugquelle gleichzeitig zu reinigen. Sobald ein Rohrschaftinstrument gereinigt ist, wird der diesem Rohrschaftinstrument zugeordnete Anschluß verschlossen, so daß mit einer solchen Vorrichtung insgesamt weitergearbeitet werden kann, während das vorgereinigte Rohrschaftinstrument abgenommen wird.

Andererseits kann bei einer abgewandelten Ausführungsform vorgesehen sein, daß man Gasblasen in das Reinigungsbad einleitet, die im Bereich der Ansaugöffnungen des Hohlraumes in der Reinigungsflüssigkeit aufsteigen und dadurch in das Rohrschaftinstrument eingesaugt werden. Durch die aufsteigenden Gasblasen, bei denen es sich vorzugsweise um Luftblasen handelt, wird eine Turbulenz in der Reinigungsflüssigkeit erzeugt, die sich auch im Inneren des Rohrschaftinstruments fortsetzt, da die Blasen mit angesaugt werden. Diese Turbulenz fördert die Reinigungswirkung der Reinigungsflüssigkeit.

Es kann weiterhin vorgesehen sein, daß man bei Rohrschaftinstrumenten mit gegeneinander beweglichen Teilen diese Teile während des Reinigungsvorganges relativ zueinander bewegt. Die Relativbewegung der zueinander beweglich angeordneten Teile des Instrumentes stellt sicher, daß eine besonders zuverlässige Reinigung erfolgt, insbesondere werden dabei auch Partikel ausgespült, die bei feststehenden Teilen möglicherweise eingeklemmt sein könnten.

Bei einer Vorrichtung der eingangs beschriebenen Art wird die genannte Aufgabe erfindungsgemäß durch die kennzeichnenden Merkmale des Patentanspruchs 5 gelöst.

Die Verschließeinrichtung kann beispielsweise ein handbetätigbares Schließventil sein. Bei einer besonderen Ausführungsform ist vorgesehen, daß die Verschließeinrichtung ein Stopfen ist, der an der Vorrichtung festgelegt ist und auf den der Schlauch mit seinem freien Ende dichtend aufsteckbar ist.

Dabei ist es günstig, wenn die Halterung aus mindestens zwei Teilen besteht, die zwischen einer Arbeitslage mit großer Bauhöhe und einer Ruhelage mit geringer Bauhöhe relativ zueinander bewegbar sind. Es ist dadurch möglich, die Halterung auch dann auf kleinem Raum aufzubewahren, wenn in ihr relativ große Instrumente gehaltert werden sollen. In der Arbeitslage ist die Halterung nämlich dann für die relativ großen Instrumente dimensionsmäßig geeignet, in der Ruhelage hingegen ergibt sich eine erhebliche Verringerung der Baugröße der Halterung.

Dabei ist es günstig, wenn die beiden Teile der Arbeitslage relativ zueinander fixierbar sind, beispielsweise durch eine Rastverbindung oder durch einen Rasthebel.

Der obere Teil kann beispielsweise gegenüber dem unteren Teil der Halterung verschwenkbar ausgebildet sein.

Bei einer bevorzugten Ausführungsform ist ein mit der Reinigungsflüssigkeit befüllbarer Behälter vorgesehen, der mittels eines Deckels verschließbar und so bemessen ist, daß er die Halterung zumindest in der Ruhelage vollständig aufnimmt. Damit kann die Gesamtvorrichtung einschließlich der Halterung in einem abgeschlossenen Behälter untergebracht werden, der beispielsweise nach Art eines Sterilisierbehälters ausgebildet ist und auch in seinen Abmessungen den Abmessungen herkömmlicher Sterilisierbehälter entspricht, obwohl in einem solchen Behälter Instrumente gereinigt werden können, die unter Umständen größere Abmessungen haben.

Günstig ist es, wenn im Behälter oberhalb des Halters ein herausnehmbarer Einsatz angeordnet ist, in den die Anschlußschläuche einlegbar sind. Damit ergibt sich in einem solchen Behälter eine vollständige Spülvorrichtung, die lediglich an eine Saugquelle angeschlossen werden muß, beispielsweise an eine in Operationsräumen ohnehin übliche Sekretabsaugung.

Bei einer bevorzugten Ausführungsform ist weiterhin vorgesehen, daß im Behälter unterhalb der in die Reinigungsflüssigkeit eintauchenden Instrumente mindestens ein Einlaß für ein Gas vorgesehen ist, durch welchen das Gas unterhalb der Instrumente in die Reinigungsflüssigkeit eintreten kann.

Eine weitere bevorzugte Ausführungsform zeichnet sich dadurch aus, daß ein an den Rohrschaftinstrumenten angreifender Antrieb vorgesehen ist, der gegeneinander bewegliche Teile der Rohrschaftinstrumente gegeneinander bewegt.

Die nachfolgende Beschreibung bevorzugter Ausführungsformen der Erfindung dient im Zusammenhang mit der Zeichnung der näheren Erläuterung:

Es zeigen:
- Fig. 1:: eine perspektivische Ansicht eines ersten bevorzugten Ausführungsbeispiels einer Reinigungsvorrichtung für ärztliche Rohrschaftinstrumente;
- Fig. 2:: eine abgewandelte Ausführungsform einer Reinigungsvorrichtung mit einer Halterung für die zu reinigenden Instrumente;
- Fig. 3:: eine Draufsicht auf die Vorrichtung der Fig. 2;
- Fig. 4: eine Querschnittsansicht längs Linie 4-4 in Fig. 3 und
- Fig. 5:: eine Querschnittsansicht eines Behälters zur Aufnahme der Vorrichtung der Fig. 2.

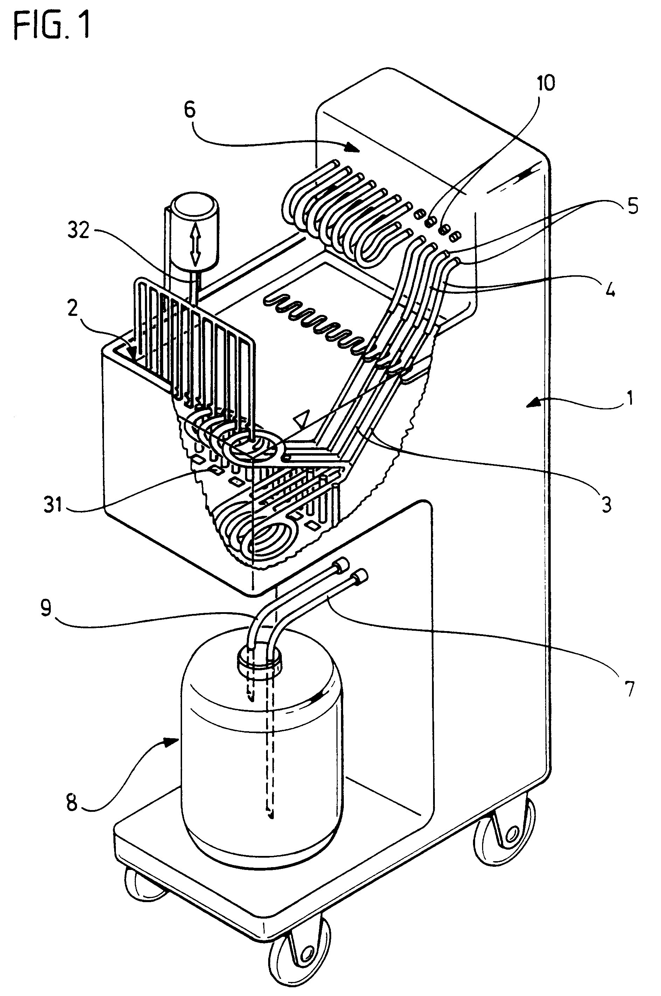

Das in Fig. 1 dargestellte Ausführungsbeispiel einer Reinigungsvorrichtung umfaßt ein fahrbares Gehäuse 1 mit einem offenen Flüssigkeitsbehälter 2, der mit einer Reinigungsflüssigkeit gefüllt ist.

Im Innern des Flüssigkeitsbehälters 2 werden an einer geeigneten, in der Zeichnung nicht dargestellten Halterung eine Vielzahl von ärztlichen Rohrschaftinstrumenten gehalten, im folgenden als Instrumente 3 bezeichnet. Diese sind so in die Flüssigkeit im Flüssigkeitsbehälter eingetaucht, daß ihr Griffteil und ein großer Teil des daran anschließenden Schaftes in die Flüssigkeit eintauchen, während das freie Ende aus der Flüssigkeit heraussteht. Auf dieses freie Ende ist jeweils ein Schlauch 4 dichtend aufgeschoben, beispielsweise ein Silikonschlauch, der jeweils zu einem Anschluß in einer oberhalb des Flüssigkeitsbehälters 2 angeordneten Konsole 6 führt. Innerhalb der Konsole ist ein in der Zeichnung nicht extra markierter Saugraum angeordnet, der über eine Leitung 7 mit einem Sammelgefäß 8 in Verbindung steht, welches unterhalb des Flüssigkeitsbehälters 2 und von vorne her frei zugänglich am Gehäuse 1 abgestellt ist. Der Innenraum des Sammelgefäßes 8 ist über eine weitere Leitung 9 mit einer im Inneren des Gehäuses 1 angeordneten und in der Zeichnung nicht dargestellten Saugpumpe verbunden.

Oberhalb jedes Anschlusses 5 ist an der Konsole 6 jeweils ein Blindstopfen 10 gehalten, auf den der jeweils zugehörige Schlauch 4 mit seinem freien Ende so aufgesteckt werden kann, daß er verschlossen ist, wenn er nicht auf ein Instrument 3 aufgesteckt ist. In der Zeichnung sind auf der linken Seite der Konsole die Schläuche 4 dichtend auf den Stopfen 10 aufgesteckt, während sie auf der rechten Seite auf Instrumente 3 aufgeschoben sind.

Am Boden des Flüssigkeitsbehälters 2 befinden sich unterhalb der Instrumente 3 Einlaßöffnungen 31, durch die über eine in der Zeichnung nicht dargestellte Vorrichtung ein Gas in den Flüssigkeitsbehälter 2 eingeführt werden kann, vorzugsweise Luft. Diese Gasblasen steigen in der Reinigungsflüssigkeit im Bereich der Ansaugöffnungen der Instrumente 3 auf und führen zu einer Turbulenz in der Reinigungsflüssigkeit.

Nur schematisch ist in Figur 1 ein motorischer Antrieb 32 dargestellt, der an allen Instrumenten angreift und, beispielsweise über einen Exzentertrieb, die beweglichen Teile der Instrumente 3 während des Reinigungsvorganges periodisch gegeneinander bewegt. Im dargestellten Ausführungsbeispiel werden die beiden Branchen der Rohrschaftinstrumente periodisch geöffnet und geschlossen.

Im Betrieb werden die zu reinigenden Instrumente in der beschriebenen Weise in die Reinigungsflüssigkeit des Flüssigkeitsbehälters 2 soweit eingetaucht, daß der größte Teil unterhalb des Flüssigkeitsspiegels angeordnet wird. Auf den nach oben aus der Flüssigkeit herausstehenden Teil werden Schläuche 4 aufgesteckt, so daß beim Einschalten der Saugpumpe im Sammelgefäß 8 ein Unterdruck erzeugt wird. Dieser führt zu einem Ansaugen der Reinigungsflüssigkeit durch das jeweilige Instrument 3 hindurch, wobei die Reinigungsflüssigkeit nicht nur durch das Schaftende eintreten kann, sondern durch alle anderen Öffnungen, die sich beispielsweise bei Gelenken oder anderen Spalten ergeben können. Diese Reinigungsflüssigkeit wird durch das Instrument 3 und den Schlauch 4 hindurch in das Sammelgefäß 8 gesaugt, so daß auf diese Weise eine gründliche Reinigung des Innenraumes des Instrumentes 3 erfolgen kann.

Die Reinigungswirkung kann einerseits dadurch verbessert werden, daß in der Reinigungsflüssigkeit durch die Zufuhr von Gasblasen Turbulenzen erzeugt werden, die sich auch im Innern des gereinigten Rohrschaftinstrumentes fortsetzen, zum anderen durch die periodische Bewegung der beweglichen Teile des Instrumentes relativ zueinander. Dadurch wird eine vollständige Umspülung aller Bereiche des Instrumentes gewährleistet.

Nach Beendigung der Reinigung wird der Schlauch 4 vom jeweiligen Instrument 3 abgezogen und auf den zugehörigen Stopfen aufgesteckt, so daß kein Nebenschluß entsteht, durch den Falschluft angesaugt wird. Die Saugleistung wird daher in vollem Umfange nur auf die jeweils angeschlossenen Instrumente 3 konzentriert.

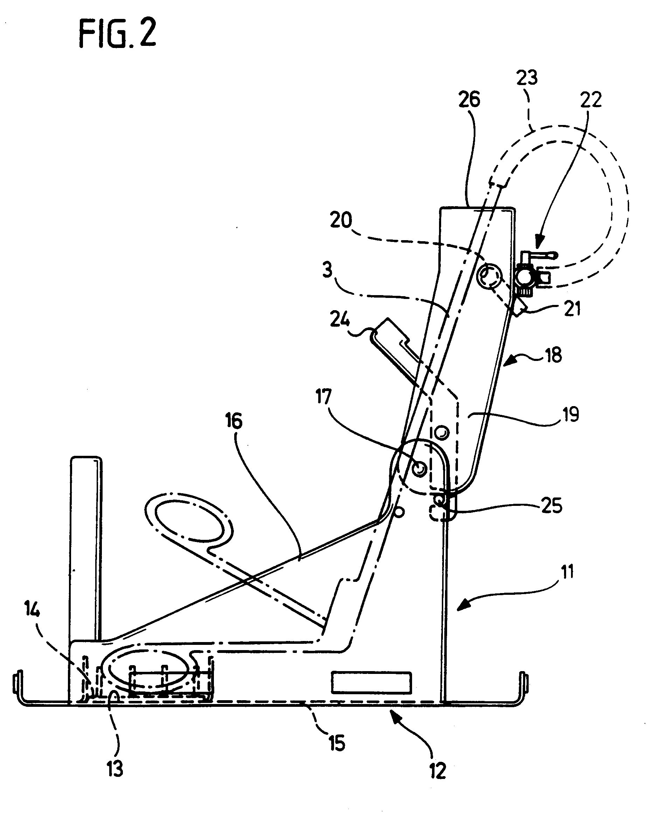

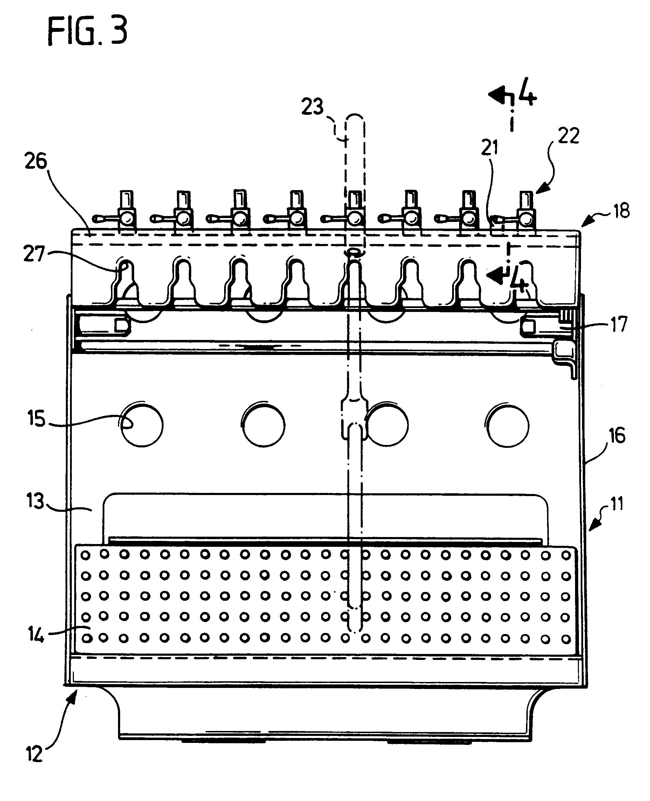

Bei dem Ausführungsbeispiel der Figuren 2 bis 5 ist eine Halterung 11 vorgesehen, die gleichzeitig auch einen Teil der Aufgaben des Gehäuses 1 beim Ausführungsbeispiel der Fig. 1 übernimmt. Die Halterung 11 umfaßt ein Unterteil 12 mit einer im wesentlichen horizontal verlaufenden Aufnahmefläche 13 für ärztliche Instrumente, die teilweise mit einer elastomeren Noppenmatte 14 belegt ist und die zusätzliche Öffnungen 15 zum Einstecken von Rohrschaftinstrumenten aufweist.

Die Aufnahmefläche 13 wird über senkrechte Seitenwangen 16 auf einem Untergrund getragen, auf dem die Halterung aufgestellt wird.

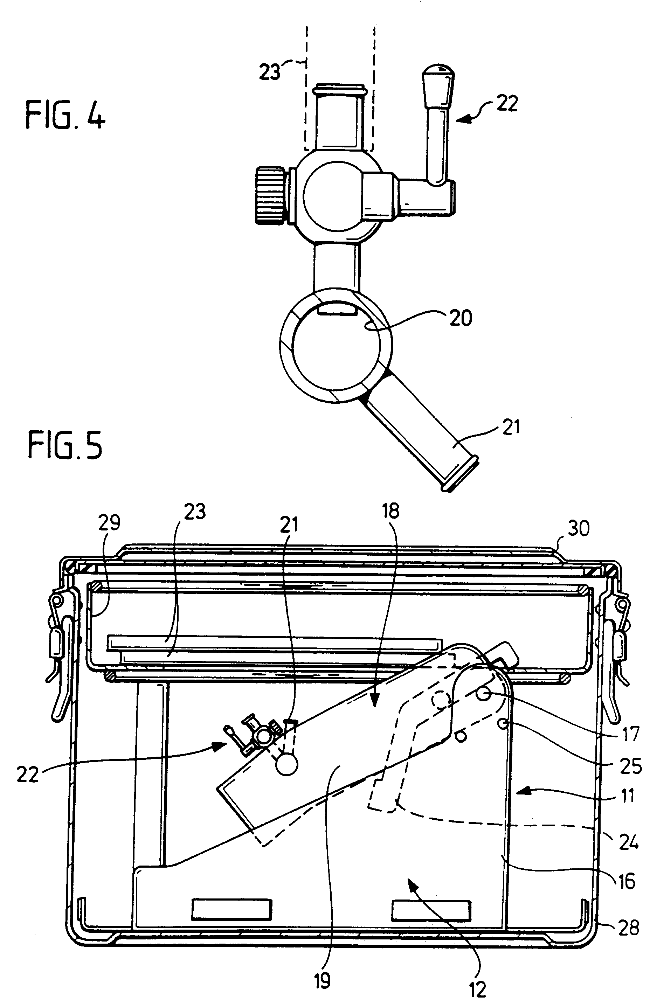

An den Seitenwangen 16 ist um eine horizontale Achse 17 verschwenkbar ein Oberteil 18 gelagert, welches an zwei parallelen Armen 19 einen sich parallel zur Achse 17 erstreckenden Saugraum 20 in Form eines sich über die gesamte Breite des Oberteiles 18 erstreckenden Rohres trägt. Dieser Saugraum 20 steht über einen Anschlußstutzen 21 (Fig. 4) mit einer Saugquelle in Verbindung, beispielsweise in gleicher Weise, wie dies bei dem Saugraum des Ausführungsbeispieles der Fig. 1 erläutert worden ist. Der Anschlußstutzen 21 kann auch mit einem herkömmlichen Sekret-Absauggerät verbunden werden, welches in Operationsräumen zum Absaugen von Körperflüssigkeiten und Körpersekreten Verwendung findet.

Der rohrförmige Saugraum 20 trägt nebeneinander eine größere Anzahl von handbetätigbaren Schließventilen 22, die jeweils mit dem Innenraum des Saugraumes 20 verbunden sind und an die jeweils ein Schlauch 23 angeschlossen ist.

Das Oberteil 18 kann nach unten in Richtung auf das Unterteil 12 verschwenkt werden, so daß sich insgesamt eine geringe Bauhöhe der Halterung 11 ergibt, diese Ruhelage ist in Fig. 5 dargestellt. Die Arbeitslage erreicht die Halterung dann, wenn das Oberteil 18 im wesentlichen senkrecht nach oben verschwenkt wird, wie dies in Fig. 2 dargestellt ist. In dieser Position wird das Oberteil 18 gegenüber dem Unterteil 12 durch einen Fixierhebel 24 festgelegt, der schwenkbar an einem Arm 19 des Oberteils 18 gelagert ist und einen Vorsprung 25 an der Seitenwange 16 des Unterteiles 12 hintergreift. Durch eine in der Zeichnung nicht dargestellte Feder wird der Fixierhebel 24 in einer Position gehalten, in der ein Verschwenken des Oberteiles 18 in die Ruhelage verhindert wird, wenn der Fixierhebel 24 unter der Wirkung der Feder in seine Endlage verschwenkt ist. Gegen die Wirkung der Feder kann der Fixierhebel 24 verschwenkt werden und gibt dann den Vorsprung 25 frei; in dieser Lage des Fixierhebels 24 kann das Oberteil 18 in die Ruhelage verschwenkt werden.

An der Stirnseite 26 des Oberteiles 18 sind mehrere Ausnehmungen 27 nebeneinander angeordnet, und zwar ist jedem Schließventil 22 eine eigene Ausnehmung 27 zugeordnet. Die Ausnehmungen 27 sind bei dem dargestellten Ausführungsbeispiel durch zur Unterseite des Oberteiles 18 hin offene, sich stufenförmig verengende Längsschlitze gebildet. In diese Längsschlitze 27 können auf die Aufnahmefläche 13 aufgesetzte Instrumente 3 eingelegt werden, so daß die Instrumente 3 an der Halterung eine definierte Position einnehmen, wobei jedem Schließventil 22 und damit jedem Schlauch 23 jeweils ein Instrument 3 zugeordnet ist.

In der Ruhelage hat die Halterung 11 eine relativ geringe Bauhöhe, so daß sie in den wannenförmigen Unterteil 28 eines kastenförmigen Behälters eingesetzt werden kann, in den oberhalb der Halterung 11 noch ein Einsatz 29 in Form eines Siebkorbes eingesetzt ist (Fig. 5). Auf dem Unterteil 28 ist abdichtend ein Deckel 30 aufgesetzt. der aus Unterteil 28 und Deckel 30 bestehende Behälter ist nach Art eines Sterilisierbehälters ausgebildet, besteht also aus Metall und ist auch in seinen Abmessungen vorzugsweise an die üblichen Abmessungen eines Sterilisierbehälters angepaßt. Der Siebkorb 29 nimmt normalerweise die Schläuche 23 auf.

Im Betrieb wird der Siebkorb 29 mit den Schläuchen aus dem Behälter herausgenommen, anschließend werden die Schläuche 23 auf die jeweiligen Schließventile 22 aufgesteckt.

Die Halterung 11 wird mit dem Oberteil 18 in Arbeitslage in das Unterteil 28 des Behälters hineingestellt, dieses Unterteil 28 wird mit der Reinigungsflüssigkeit gefüllt. Vor der Befüllung oder danach werden die zu reinigenden Instrumente in die Halterung 11 eingesetzt, wobei sie auf der Noppenmatte 14 oder in den Öffnungen 15 einerseits und in den Ausnehmungen 27 andererseits festgelegt werden. Auf die aus der Flüssigkeit herausstehenden Enden der Instrumente 3 werden die Schläuche 23 aufgesteckt. Nur bei den aufgesteckten Schläuchen werden anschließend die Schließventile 22 manuell geöffnet, so daß über den Anschlußstutzen 21 und die daran angeschlossene Vakuumquelle Reinigungsflüssigkeit durch die Instrumente 3 in den Saugraum 20 hindurch angesaugt wird.

Der in Fig. 5 dargestellte Behälter mit der eingesetzten Halterung nimmt somit alle für die Reinigung der Instrumente notwendigen Bestandteile auf und kann gleichzeitig als Reinigungsbehälter verwendet werden, es ist lediglich notwendig, über den Anschlußstutzen 21 die gesamte Vorrichtung an eine herkömmliche Vakuumquelle anzuschließen.

## Patentansprüche

1. Verfahren zur Reinigung ärztlicher Rohrschaftinstrumente, bei dem man die Rohrschaftinstrumente mit einem Ende in eine Reinigungsflüssigkeit eintaucht und am anderen Ende Reinigungsflüssigkeit absaugt, dadurch gekennzeichnet, daß man die Rohrschaftinstrumente mit ihrem Griffteil und einem großen Teil des daran anschließenden Schafts in die Reinigungsflüssigkeit eintaucht, während das freie Ende aus der Flüssigkeit heraussteht, und daß man auf das freie Ende des Rohrschaftinstruments einen Absaugschlauch dichtend aufschiebt.

2. Verfahren nach Anspruch 1, dadurch gekennzeichnet, daß man mehrere Rohrschaftinstrumente über jeweils einen Saugschlauch gleichzeitig an eine gemeinsame Saugleitung anschließt und jeden Anschluß zwischen einem Rohrschaftinstrument und der Saugleitung individuell öffnet oder schließt.

3. Verfahren nach einem der Ansprüche 1 oder 2, dadurch gekennzeichnet, daß man Gasblasen in das Reinigungsbad einleitet, die im Bereich der Ansaugöffnungen des Rohrschaftinstruments in der Reinigungsflüssigkeit aufsteigen und dadurch in das Rohrschaftinstrument eingesaugt werden.

4. Verfahren nach einem der Ansprüche 1 bis 3, dadurch gekennzeichnet, daß man bei Rohrschaftinstrumenten mit gegeneinander beweglichen Teilen diese Teile während des Reinigungsvorganges relativ zueinander bewegt.

5. Vorrichtung zur Reinigung ärztlicher Rohrschaftinstrumente (3) mit einem Behälter (2; 28) für eine Reinigungsflüssigkeit, in die die Rohrschaftinstrumente (3) eintauchen, dadurch gekennzeichnet, daß eine Halterung (11) vorgesehen ist, an der die Rohrschaftinstrumente (3) so positionierbar sind, daß sie mit ihrem Griffteil und einem großen Teil des daran anschließenden Schafts in die Reinigungsflüssigkeit eintauchen, daß am oberen Teil (18) der Halterung (11) ein Saugraum (20) angeordnet ist, der nebeneinander eine Anzahl von Verschließeinrichtungen (10; 22) trägt, und daß sich an jede Verschließeinrichtung (10; 22) als Anschluß an die Rohrschaftinstrumente (3) ein auf das freie Ende des Schafts dichtend aufsteckbarer Schlauch (4; 23) anschließt.

6. Vorrichtung nach Anspruch 5, dadurch gekennzeichnet, daß die Verschließeinrichtung (22) ein handbetätigbares Schließventil ist.

7. Vorrichtung nach Anspruch 5, dadurch gekennzeichnet, daß die Verschließeinrichtung (10) ein Stopfen ist, der an der Vorrichtung festgelegt ist und auf den der Schlauch (4) mit seinem freien Ende dichtend aufsteckbar ist.

8. Vorrichtung nach einem der Ansprüche 5 bis 7, dadurch gekennzeichnet, daß die Halterung (11) aus mindestens zwei Teilen (12, 18) besteht, die zwischen einer Arbeitslage mit großer Bauhöhe und einer Ruhelage mit geringer Bauhöhe relativ zueinander bewegbar sind.

9. Vorrichtung nach Anspruch 8, dadurch gekennzeichnet, daß die beiden Teile (12, 18) in der Arbeitslage relativ zueinander fixierbar sind.

10. Vorrichtung nach Anspruch 8 oder 9, dadurch gekennzeichnet, daß der obere Teil (18) gegenüber dem unteren Teil (12) der Halterung (11) verschwenkbar ist.

11. Vorrichtung nach einem der Ansprüche 5 bis 10, dadurch gekennzeichnet, daß ein mit der Reinigungsflüssigkeit befüllbarer Behälter (28) vorgesehen ist, der mittels eines Deckels (30) verschließbar und so bemessen ist, daß er die Halterung (11) zumindest in der Ruhelage vollständig aufnimmt.

12. Vorrichtung nach Anspruch 11, dadurch gekennzeichnet, daß im Behälter (28) oberhalb der Halterung (11) ein herausnehmbarer Einsatz (29) angeordnet ist, in den die Anschlußschläuche (23) einlegbar sind.

13. Vorrichtung nach einem der Ansprüche 5 bis 12, dadurch gekennzeichnet, daß im Behälter unterhalb der in die Reinigungsflüssigkeit eintauchenden Rohrschaftinstrumente (3) mindestens ein Einlaß (31) für ein Gas vorgesehen ist, durch welchen das Gas unterhalb der Rohrschaftinstrumente (3) in die Reinigungsflüssigkeit eintreten kann.

14. Vorrichtung nach einem der Ansprüche 5 bis 13, dadurch gekennzeichnet, daß ein an den Rohrschaftinstrumenten (3) angreifender Antrieb (32) vorgesehen ist, der gegeneinander bewegliche Teile der Rohrschaftinstrumente (3) gegeneinander bewegt.

## Claims

1. A method of cleaning medical tubular-shafted instruments, in which one end of the tubular-shafted instruments is immersed in a cleaning fluid and cleaning fluid is drawn off at the other end, characterised in that the handle of the tubular-shafted instruments and a large part of the shaft joined thereto are immersed in the cleaning fluid, while the free end of the tubular-shafted instruments projects from the fluid, and in that a suction hose is sealingly pushed onto the free end of the tubular-shafted instrument.

2. A method in accordance with Claim 1, characterised in that a plurality of tubular-shafted instruments are each connected at the same time to a common suction line via a respective suction hose, and each connection between a tubular-shafted instrument and the suction line is opened or closed individually.

3. A method in accordance with either of Claims 1 or 2, characterised in that gas bubbles are introduced into the cleaning bath, these gas bubbles rising in the cleaning fluid in the region of the suction openings of the tubular-shafted instrument and thereby being drawn into the tubular-shafted instrument.

4. A method in accordance with any one of Claims 1 to 3, characterised in that with tubular-shafted instruments having parts movable in relation to one another, these parts are moved relative to one another during the cleaning procedure.

5. A device for cleaning medical tubular-shafted instruments (3), having a container (2; 28) for a cleaning fluid in which the tubular-shafted instruments (3) are immersed, characterised in that a holding device (11) is provided on which the tubular-shafted instruments (3) can be positioned in such a manner that their handle and a large part of the shaft joined thereto are immersed in the cleaning fluid, in that a suction chamber (20) is arranged at the upper part (18) of the holding device (11) and bears a number of sealing devices (10; 22) arranged side by side, and in that a hose (4; 23) sealingly attachable to the free end of the shaft is joined to each sealing device (10; 22) as a connection to the tubular-shafted instruments (3).

6. A device in accordance with Claim 5, characterised in that the sealing device (22) is a manually operable closing valve.

7. A device in accordance with Claim 5, characterised in that the sealing device (10) is a stopper which is secured to the device and to which the free end of the hose (4) is sealingly attachable.

8. A device in accordance with any one of Claims 5 to 7, characterised in that the holding device (11) is composed of at least two parts (12, 18) movable relative to one another between an operative position with great over-all height and a rest position with low over-all height.

9. A device in accordance with Claim 8, characterised in that in the operative position, the two parts (12, 18) can be fastened relative to one another.

10. A device in accordance with Claim 8 or 9, characterised in that the upper part (18) is pivotable relative to the lower part (12) of the holding device (11).

11. A device in accordance with any one of Claims 5 to 10, characterised in that a container (28) which can be filled with the cleaning fluid is provided, this container (28) being sealable by means of a cover (30) and being dimensioned such that in the rest position at least, it receives the entire holding device (11).

12. A device in accordance with Claim 11, characterised in that a removable insert (29) is arranged in the container (28) and is located above the holding device (11) therein, the connecting hoses (23) being able to be placed in this insert (29).

13. A device in accordance with any one of Claims 5 to 12, characterised in that at least one inlet (31) for a gas is provided in the container and is located below the tubular-shafted instruments (3) therein which are immersed in the cleaning fluid, the gas being able to enter into the cleaning fluid below the tubular-shafted instruments (3) through this inlet (31).

14. A device in accordance with any one of Claims 5 to 13, characterised in that a drive (32) acting upon the tubular-shafted instruments (3) is provided and moves in relation to one another those parts of the tubular-shafted instruments (3) movable in relation to one another.

## Revendications

1. Procédé pour nettoyer des instruments médicaux à tige tubulaire, dans lequel on plonge les instruments à tige tubulaire par une extrémité dans un liquide de nettoyage et on aspire le liquide de nettoyage à l'autre extrémité, caractérisé en ce que l'on plonge les instruments à tige tubulaire dans le liquide de nettoyage par leur partie de poignée et par me grande partie de la tige qui s'y raccorde, tandis que l'extrémité libre dépasse hors du liquide, et en ce que l'on enfiche avec étanchement un tuyau d'aspiration sur l'extrémité libre de l'instrument à tige tubulaire.

2. Procédé selon la revendication 1, caractérisé en ce que l'on raccorde simultanément plusieurs instruments à tige tubulaire via un tuyau d'aspiration respectif à me conduite d'aspiration commune, et en ce que l'on ouvre ou ferme individuellement chaque raccordement entre un instrument à tige tubulaire et la conduite d'aspiration.

3. Procédé selon l'une ou l'autre des revendications 1 et 2, caractérisé en ce que l'on introduit des bulles de gaz dans le bain de nettoyage, qui montent dans la région des ouvertures d'aspiration de l'instrument à tige tubulaire dans le liquide de nettoyage et sont ainsi aspirées dans l'instrument à tige tubulaire.

4. Procédé selon l'une quelconque des revendications 1 à 3, caractérisé en ce que dans des instruments à tige tubulaire comportant des éléments mobiles les uns par rapport aux autres, ces éléments sont déplacés les uns par rapport aux autres pendant l'opération de nettoyage.

5. Dispositif pour nettoyer des instruments à tige tubulaire médicaux (3) comportant un récipient (2 ; 28) pour un liquide de nettoyage dans lequel plongent les instruments à tige tubulaire (3), caractérisé en ce qu'il est prévu une monture (11) sur laquelle les instruments à tige tubulaire (3) peuvent être positionnés de telle sorte qu'ils plongent par leur partie de poignée et par une grande partie de la tige qui s'y raccorde dans le liquide de nettoyage, en ce qu'il est prévu sur la partie supérieure (18) de la monture (11) une chambre d'aspiration (20) qui porte un certain nombre de dispositifs de fermeture (10 ; 22) agencés les uns à côté des autres, et en ce qu'un tuyau (4 ; 23) enfichable avec étanchement sur l'extrémité libre de la tige se raccorde à chaque dispositif de fermeture (10 ; 22) en tant que raccordement aux instruments à tige tubulaire (3).

6. Dispositif selon la revendication 5, caractérisé en ce que le dispositif de fermeture (22) est une vanne de fermeture actionnée à la main.

7. Dispositif selon la revendication 5, caractérisé en ce que le dispositif de fermeture (10) est un bouchon qui est fixé sur le dispositif et sur lequel le tuyau (4) est enfichable avec étanchement par son extrémité libre.

8. Dispositif selon l'une quelconque des revendications 5 à 7, caractérisé en ce que la monture (11) est constituée par au moins deux parties (12, 18) qui sont mobiles l'une par rapport à l'autre entre une position de travail présentant une hauteur structurelle élevée et une position de repos présentant une hauteur structurelle faible.

9. Dispositif selon la revendication 8, caractérisé en ce que les deux parties (12, 18) peuvent être fixées l'une par rapport à l'autre dans la position de travail.

10. Dispositif selon les revendications 8 ou 9, caractérisé en ce que la partie supérieure (18) peut être basculée par rapport à la partie inférieure (12) de la monture (11).

11. Dispositif selon l'une quelconque des revendications 5 à 10, caractérisé en ce qu'il est prévu un récipient (28) susceptible d'être rempli avec le liquide de nettoyage, qui peut être fermé au moyen d'un couvercle (30) et qui est dimensionné de telle sorte qu'il loge entièrement la monture (11), du moins dans la position de repos.

12. Dispositif selon la revendication 11, caractérisé en ce qu'il est prévu dans le récipient (28) au-dessus de la monture (11) un insert (29) extractible, dans lequel les tuyaux de raccordement (23) peuvent être introduits.

13. Dispositif selon l'une quelconque des revendications 5 à 12, caractérisé en ce qu'il est prévu dans le récipient au-dessous des instruments à tige tubulaire (3) plongeant dans le liquide de nettoyage, au moins une admission (31) pour un gaz, à travers laquelle le gaz peut entrer au-dessous des instruments à tige tubulaire (3) jusque dans le liquide de nettoyage.

14. Dispositif selon l'une quelconque des revendications 5 à 13, caractérisé en ce qu'il est prévu un entramement (32) qui attaque les instruments à tige tubulaire (3), et qui déplace les unes par rapport aux autres des parties mutuellement mobiles des instruments à tige tubulaire (3).
